# EUROPÄISCHE PATENTSCHRIFT

(11) **EP 4 037 909 B1**
(45) Veröffentlichungstag und Bekanntmachung des Hinweises auf die Patenterteilung: **29.03.2023**
(21) Anmeldenummer: 20816965.6
(22) Anmeldetag: 30.11.2020
(51) Int. Cl.: B41M 5/333, C07C 317/42

(54) **POLYMORPHE FORMEN VON N-(4-((4-(3-PHENYLUREIDO)PHENYL)SULFONYL)PHENYL)BENZOLSULFONAMID UND WÄRMEEMPFINDLICHE AUFZEICHNUNGSMATERIALIEN WELCHE DIESE ENTHALTEN**
POLYMORPHIC FORMS OF N-(4-((4-(3-PHENYLUREIDO)PHENYL)SULFONYL)PHENYL)BENZOLSULFONAMIDE AND HEAT SENSITIVE RECORDING MEDIA CONTAINING THE SAME
FORMES POLYMORPHES DE N-(4-((4-(3-PHÉNYLURÉIDO)PHÉNYLE)SULFONYLE)PHÉNYLE)BENZOLSULFONAMIDE ET MATÉRIAUX THERMOSENSIBLES LES CONTENANT

(30) Priorität: 28.11.2019 DE 102019132401
(43) Veröffentlichungstag der Anmeldung: 10.08.2022
(73) Patentinhaber: Koehler Paper SE, 77704 Oberkirch (DE)
(72) Erfinder: HORN, Michael, 77654 Offenburg (DE); STALLING, Timo, 77767 Appenweier (DE); SCHNICK, Christian, 79249 Merzhausen (DE)
(74) Vertreter: Held, Stephan
(86) Internationale Anmeldenummer: PCT/EP2020/083901
(87) Internationale Veröffentlichungsnummer: WO 2021/105499

(56) Entgegenhaltungen:
- EP-A1- 2 923 851
- EP-A1- 3 263 553

## Beschreibung

Die Erfindung betrifft polymorphe Formen von *N*-(4-((4-(3-Phenylureido)phenyl)sulfonyl)phenyl) benzolsulfonamid, Verfahren zu deren Herstellung, ihre Verwendung als Farbentwickler in wärmeempfindlichen Aufzeichnungsmaterialien, wärmeempfindliche Aufzeichnungsmaterialien, umfassend die polymorphen Formen von *N*-(4-((4-(3-Phenylureido)phenyl)sulfonyl)phenyl) benzolsulfonamid und ein Verfahren zur Herstellung solcher wärmeempfindlicher Aufzeichnungsmaterialien.

Wärmeempfindliche Aufzeichnungsmaterialien für die Thermodirektdruckanwendung, die eine auf einem Trägersubstrat aufgebrachte wärmeempfindliche farbbildende Schicht (Thermoreaktionsschicht) aufweisen, sind seit langem bekannt. In der wärmeempfindlichen farbbildenden Schicht liegen üblicherweise ein Farbbildner und ein Farbentwickler vor, die unter Wärmeeinwirkung miteinander reagieren und so zu einer Farbentwicklung führen. Benzolsulfonamide und ihre Verwendung in wärmeempfindlichen Aufzeichnungsmaterialien sind beispielsweise aus der EP2923851 A1 bekannt.

Die (nachveröffentlichte) DE102018133168 (auch veröffentlicht als WO2020/127675A1, Stand der Technik nach Art. 54(3) EPÜ) beschreibt ein wärmeempfindliches Aufzeichnungsmaterial, umfassend ein Trägersubstrat sowie eine mindestens einen Farbbildner und mindestens einen phenolfreien Farbentwickler enthaltende wärmeempfindliche farbbildende Schicht sowie die Verwendung des im wärmeempfindlichen Aufzeichnungsmaterial enthaltenen phenolfreien Farbentwicklers, wobei einer der offenbarten Farbentwickler *N*-(4-((4-(3-Phenylureido)phenyl)sulfonyl)phenyl)benzolsulfonamid, dargestellt durch Formel (1), 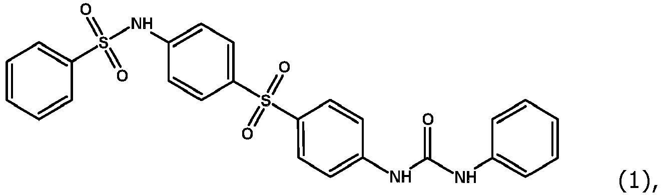 ist.

Obwohl die in der DE102018133168 beschriebene kristalline Verbindung (1) brauchbare Eigenschaften im Hinblick auf ihre Verwendung als Farbentwickler in wärmeempfindlichen Aufzeichnungsmaterialien aufweist, besteht die Notwendigkeit, gelegentlich auftretenden Schwankungen in manchen anwendungstechnischen Eigenschaften der mit der Verbindung (1) hergestellten wärmeempfindlichen Aufzeichnungsmaterialien, welche in der Regel im Zusammenhang mit Änderungen der Aufreinigungsbedingungen bei der Synthese oder im Zuge der Syntheseoptimierung von der Verbindung (1) stehen und nicht durch schwankende Reinheitsgrade von Verbindung (1) erklärbar sind, zu eruieren und zu beheben. Dies ist insbesondere vor dem Hintergrund der Verwendung der Verbindung (1) als Farbentwickler für die Herstellung wärmeempfindlicher Aufzeichnungsmaterialien im großtechnischen Maßstabe von großer Wichtigkeit, da die Spezifikationstreue der eingesetzten Rohstoffe eine wesentliche Anforderung darstellt, welche vom Herstellungsprozess des Farbentwicklers sichergestellt werden muss.

Die entsprechend der DE102018133168 hergestellten wärmeempfindlichen Aufzeichnungsmaterialien zeichnen sich durch die Ausgewogenheit wichtiger anwendungstechnischer Eigenschaften aus. Dessen ungeachtet, ist es erstrebenswert, einzelne Eigenschaften zu verbessern, ohne andere nachteilig zu beeinflussen.

Moleküle phenolfreier Farbentwickler wie die Verbindung (1) haben dank ihrer diversen funktionellen Gruppen vielfältige intra- und intermolekulare Bindungsmöglichkeiten im Kristall, welche zum Auftreten vielfältiger Kristallstrukturen (polymorphe Modifikationen) bei diesen kristallinen Verbindungen führen können. Ein wichtiger Aspekt bei der Charakterisierung solcher Stoffe durch ihre physikalisch-chemischen Eigenschaften betrifft die Kenntnis der Kristallstruktur-Landschaft. Dies beinhaltet im Wesentlichen die Identifizierung polymorpher Modifikationen und Kenntnisse über deren Identität. Eine Aufgabe der vorliegenden Erfindung ist es daher, neue phasenreine polymorphe Formen sowie Verfahren zu deren Herstellung bereitzustellen.

Eine weitere Aufgabe der vorliegenden Erfindung ist es, Optimierungspotenziale für die anwendungstechnischen Eigenschaften von mit unterschiedlichen polymorphen Formen erhaltenen wärmeempfindlichen Aufzeichnungsmaterialien gegenüber dem Stande der Technik zu identifizieren und zu nutzen.

Insbesondere besteht eine weitere Aufgabe der vorliegenden Erfindung darin, kristalline Formen eines Farbentwicklers und diese enthaltende wärmeempfindliche Aufzeichnungsmaterialien bereitzustellen, welches eine signifikant niedrigere statische Sensitivität (höhere Starttemperatur der farbbildenden Reaktion) im Vergleich zu den entsprechenden wärmeempfindlichen Aufzeichnungsmaterialien des Standes der Technik aufweisen, ohne anderweitige anwendungstechnische Nachteile zu zeigen, insbesondere ohne Einbußen der dynamischen Sensitivität (dynamische Ansprechempfindlichkeit im Druckprozess).

Polymorphe Formen von gängigen phenolfreien Farbentwickler und deren Einsatz in wärmeempfindlichen Aufzeichnungsmaterialien sind bekannt.

Die WO03/101943 A1 offenbart drei polymorphe Formen des phenolfreien Farbentwicklers Pergafast^{®} 201 (BASF), deren Herstellung, Umwandlung und Verwendung in wärmeempfindlichen Aufzeichnungsmaterialien.

Die EP3263553 A1 offenbart eine neue polymorphe Form des Farbentwicklers N-(2-(3-Phenylureido)phenyl)benzolsulfonamid und deren Verwendung in wärmeempfindlichen Aufzeichnungsmaterialien.

In der JP3991857 B werden polymorphe Formen von *n*-Butyl 4-(3-(p-toluolsulfonyl)ureido)benzoat und deren vorteilhafte Verwendung als Farbentwickler in wärmeempfindlichen Aufzeichnungsmaterialien offenbart.

Den Erfindern ist es gelungen von der in der DE102018133168 beschriebenen Verbindung *N*-(4-((4-(3-Phenylureido)phenyl)sulfonyl)phenyl)benzolsulfonamid (1) die polymorphe Form ω^{18,9}, charakterisiert durch ein Röntgenpulverdiffraktogramm mit den Braggschen Winkeln (2θ/CuK_{α}): 10,9, 11.7, 14,6, 15,0, 15,8, 16,6, 17,6, 18,9, 19,4, 20,9, 21,2, 22,0, 23,3, 24,4, 24,7, 26,1, 27,4, 29,4, 34,2 und die polymorphe Form α^{21,2} charakterisiert durch ein Röntgenpulverdiffraktogramm mit den Braggschen Winkeln (2θ/CuK_{α}): 8,7, 9,8, 10,8, 13,2, 13,9, 14,9, 15,2, 16,0, 17,4, 17,7, 18,7, 20,4, 21,2, 21,6, 22,3, 23,0, 23,3, 23,9, 24,4, 25,0, 25,8, 26,6, 28,1, 28,9, 29,4, 30,1, 30,6, 31,8, 34,5, 35,3, 35,6, 36,9 bereitzustellen.

Bei den als ω^{18,9} und α^{21,2} bezeichneten Kristallformen bezieht sich die hochgestellte Zahl auf den intensivsten Hauptpeak in dem jeweiligen Röntgenpulverdiffraktogramm.

Diese neuen polymorphen Formen sind ferner durch ihre Schmelzbereiche und ihre IR-Absorptionsbanden gemäß der folgenden Tabelle 1 charakterisiert.

Tabelle 1 fasst die wichtigsten messtechnischen Daten der erfindungsgemäßen polymorphen Formen von *N*-(4-((4-(3-Phenylureido)phenyl)sulfonyl)phenyl)benzolsulfonamid, sofern sie die Reflexe aus den P-XRD-Diffraktogrammen, charakteristische FTIR-Banden sowie das Schmelzverhalten (DSC) betreffen, zusammen.

**Tabelle 1**

| Polymorph | 2 θ-Werte der intensivsten XRD-Peaks^{#} | Schmelzbereich (°C), Onset (DSC)* | Charakteristische IR-Banden (cm⁻¹)^{∗} |
|---|---|---|---|
| ω^{18,9} | 10,9, 11,7, 14,6, 15,0, 15,8, 16,6, 17,6, 18,9, 19,4, 20,9, 21,2, 22,0, 23,3, 24,4, 24,7, 26,1, 27,4, 29,4, 34,2 | 232-233 | 1092 (m), 1105 (i), 1145 (i); 1233 (i); 1319 (i); 1495 (i); 1548 (i); 1598 (i); 1655 (i); 3239 (m) |
| α^{21,2} | 8,7, 9,8, 10,8, 13,2, 13,9, 14,9, 15,2, 16,0, 17,4, 17,7, 18,7, 20,4, 21,2, 21,6, 22,3, 23,0, 23,3, 23,9, 24,4, 25,0, 25,8, 26,6, 28,1, 28,9, 29,4, 30,1, 30,6, 31,8, 34,5, 35,3, 35,6, 36,9 | 219-221 | 1089 (m); 1110 (m); 1149 (i); 1237 (m); 1311 (i); 1321 (i); 1500 (i); 1556 (i); 1597 (i); 1697 (i); 3365 (m); 3408 (m) |

| | | | |
|---|---|---|---|
| ^{#} XRD, Bruker D2 Phaser; Auswahl an 2 θ-Werten mit rel. Intensität (I_{Peak}/I_{Hauptpeak}) ≥10%; Hauptpeak unterstrichen. ^{≠} Netsch DSC 200 F3 Maia^{®} Gerät, Al-Tiegel mit kaltverschweißtem, geschlossenem Deckel, Heizrate 10 K/min, 25 °C - 350 °C unter N₂-Atmosphäre. ^{∗} FTIR, KBr-Presslinge; i = intensiv, m = mittel. | | | |

Diese neuen polymorphen Formen können in phasenreiner Form durch Versetzen von nicht phasenreinem *N*-(4-((4-(3-Phenylureido)phenyl)sulfonyl)phenyl)benzolsulfonamid, hergestellt gemäß Beispiel 1, nach Umkristallisation aus EtOAc/n-Hexan (α^{21,2}) und durch Suspension in einem Lösungsmittel für einen Zeitraum von etwa 1 bis 20 Stunden bei einer Temperatur von etwa 0 °C bis zur Siedetemperatur des jeweiligen Lösungsmittels erhalten werden (ω^{18,9}).

Das Lösungsmittel kann aus der Klasse aromatischer Kohlenwasserstoffe, chlorierter aromatischer Kohlenwasserstoffe, aliphatischer oder alicyclischer Kohlenwasserstoffe, chlorierter Kohlenwasserstoffe, Dialkylacylamide, aliphatischer Ester, aliphatischer Ketone, alicyclischer Ketone, aliphatischer Ether, cyclischer Ether, aliphatischer Alkohole, alicyclische Alkohole, Alkylnitrile oder deren Gemische ausgewählt werden. Bevorzugt sind Toluol, Cyclohexan, Chloroform, Dichlormethan, Tetrachlorkohlenstoff, Chlorbenzol, Dimethylformamid, Dimethylacetamid, Ethylacetat, Aceton, Butanon, Cyclohexan, Diethylether, Tetrahydrofuran, Dioxan, 1,2-Dimethoxyethan, Nitromethan, Methanol, Ethanol, Isopropanol, Acetonitril oder deren Gemische.

Die α^{21,2}-Form kann durch Umkristallisieren aus EtOAc/n-Hexan von nicht phasenreinem N-(4-((4-(3-Phenylureido)phenyl)sulfonyl)phenyl)benzolsulfonamid, hergestellt gemäß Beispiel 1, erhalten werden.

Die ω^{18,9}-Form kann durch Suspendieren der α^{21,2}-Form in einem organischen Lösungsmittel, vorzugsweise Ethylacetat und erhitzen auf eine Temperatur von etwa 0°C bis zum Siedepunkt des Lösungsmittels für vorzugsweise etwa 1 Stunde bis 20 Stunden erhalten werden.

Die ω^{18,9}-Form kann durch Suspendieren der α^{21,2} -Form in Ethylacetat und Refluxieren der Suspension für etwa 2 Stunden bis 8 Stunden, vorzugsweise für etwa 6 Stunden, erhalten werden.

Die α^{21,2}-Form kann wiederum auch durch Umkristallisieren der phasenreinen ω^{18,9}-Form aus Acetonitril gewonnen werden.

Die vorliegende Erfindung betrifft auch *N*-(4-((4-(3-Phenylureido)phenyl)sulfonyl)phenyl)benzolsulfonamid in der polymorphen Form ω^{18,9}, erhältlich gemäß den vorstehend beschriebenen Verfahren.

Die vorliegende Erfindung betrifft auch *N*-(4-((4-(3-Phenylureido)phenyl)sulfonyl)phenyl)benzolsulfonamid in der polymorphen Form α^{21,2}, erhältlich gemäß den vorstehend beschriebenen Verfahren.

Die vorliegende Erfindung betrifft auch die Verwendung von *N*-(4-((4-(3-Phenylureido)phenyl)sulfonyl)phenyl)benzolsulfonamid in der polymorphen Form ω^{18,9} oder in der polymorphen Form α^{21,2}, wie vorstehend beschrieben oder wie erhältlich durch eines der vorstehend beschriebenen Verfahren als Farbentwickler in einem wärmeempfindlichen Aufzeichnungsmaterial.

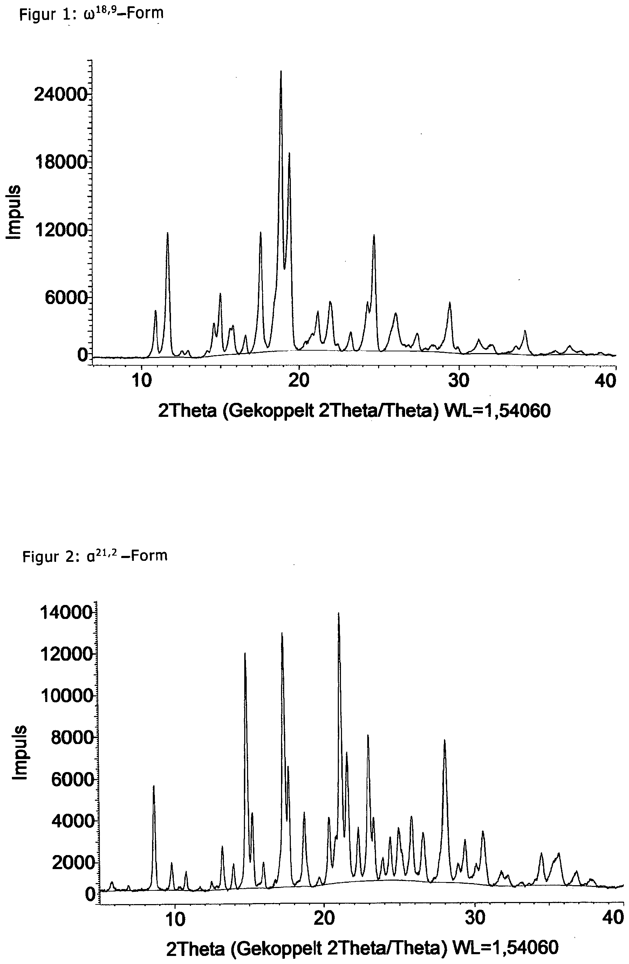

Die vorliegende Erfindung betrifft zudem ein wärmeempfindliches Aufzeichnungsmaterial, umfassend ein Trägersubstrat sowie eine mindestens einen Farbbildner und einen Farbentwickler enthaltende wärmeempfindliche farbbildende Schicht, wobei der Farbentwickler die phasenreine ω^{18,9}-Form von N-(4-((4-(3-Phenylureido)phenyl)sulfonyl)phenyl)benzolsulfonamid, charakterisiert durch ein Röntgenpulverdiffraktogramm mit folgenden Braggschen Winkeln (2θ/CuK_{α}): 10,9, 11,7, 14,6, 15,0, 15,8, 16,6, 17,6, 18,9, 19,4, 20,9, 21,2, 22,0, 23,3, 24,4, 24,7, 26,1, 27,4, 29,4, 34,2 (s. Fig. 1) oder die phasenreine α^{21,2}-Form von *N*-(4-((4-(3-Phenylureido)phenyl)sulfonyl)phenyl)benzolsulfonamid, charakterisiert durch ein Röntgenpulverdiffraktogramm mit folgenden Braggschen Winkeln (2θ/CuK_{α}): 8,7, 9,8, 10,8, 13,2, 13,9, 14,9, 15,2, 16,0, 17,4, 17,7, 18,7, 20,4, 21,2, 21,6, 22,3, 23,0, 23,3, 23,9, 24,4, 25,0, 25,8, 26,6, 28,1, 28,9, 29,4, 30,1, 30,6, 31,8, 34,5, 35,3, 35,6, 36,9 (s. Fig. 2), oder deren Gemische, umfasst.

In einer weiteren bevorzugten Ausführungsform des wärmeempfindlichen Aufzeichnungsmaterials umfasst der mindestens eine Farbentwickler die einzelnen oben beschriebenen polymorphen Formen oder deren Gemische, jeweils in Kombination mit mindestens einer Verbindung der Formel (2)

Ar¹-SO₂-NH-C₆H₄-SO₂-C₆H₄-NH-CO-NH-Ar² (2),

wobei Ar¹ und Ar² ein unsubstituierter oder substituierter Phenyl-Rest ist.

Vorzugsweise ist Ar¹ ein Phenyl-Rest.

Vorzugsweise ist Ar² ein Phenyl-Rest.

Besonders bevorzugt ist Ar¹ mit mindestens einem C₁-C₅-Alkyl-, einem Alkenyl-, einem Alkinyl-, einem Benzyl-, einem Formyl-, einem CN-, einem Halogen-, einem NO₂⁻, einem RO-, einem R-CO-, einem RO₂C-, einem R-OCO-, einem R-SO₂O-, einem R-O-SO₂-, einem R-SO₂-NH-, einem R-NH-SO₂-, einem R-NH-CO- oder einem R-CO-NH-Rest, wobei Rein C₁-C₅-Alkyl-, ein Alkenyl-, ein Alkinyl-, ein Phenyl-, ein Tolyl- oder ein Benzyl-Rest ist, substituiert.

Vorzugsweise ist Ar¹ mit mindestens einem C₁-C₅-Alkyl-, einem Halogen-, einem RO-, einem R-CO- oder einem NO₂-Rest, wobei Rein C₁-C₅-Alklyl-Rest ist, substituiert.

Vorzugsweise ist Ar¹ einfach substituiert.

Besonders bevorzugt ist Ar² mit mindestens einem C₁-C₅-Alkyl-, einem Alkenyl-, einem Alkinyl-, einem Benzyl-, einem Formyl-, einem CN-, einem Halogen-, einem NO₂₋, einem RO-, einem R-CO-, einem RO₂C-, einem R-OCO-, einem R-SO₂O-, einem R-O-SO₂-, einem R-SO₂-NH-, einem R-NH-SO₂- einem R-NH-CO- oder einem R-CO-NH-Rest, wobei Rein C₁-C₅-Alkyl-, ein Alkenyl-, ein Alkinyl-, ein Phenyl-, ein Tolyl- oder ein Benzyl-Rest ist, substituiert.

Vorzugsweise ist Ar² mit mindestens einem C₁-C₅-Alkyl-, einem Halogen-, einem RO-, einem R-CO-, einem RO₂C- oder einem NO₂-Rest, wobei Rein C₁-C₅-Alklyl-Rest ist, substituiert.

Vorzugsweise ist Ar² einfach substituiert.

Die Substitution von Ar¹ und Ar² mit mindestens einem C₁-C₅-Alkyl-Rest erfolgt vorzugsweise derart, dass der C₁-C₅-Alkyl-Rest ein Methyl- oder Butyl-Rest, besonders bevorzugt ein Methyl-Rest, ist.

Die Substitution von Ar¹ und Ar² mit mindestens einem Halogen-Rest erfolgt vorzugsweise derart, dass der Halogen-Rest ein Chlorid-Rest ist.

Die Substitution von Ar¹ und Ar² mit mindestens einem RO-Rest erfolgt vorzugsweise derart, dass der RO-Rest ein CH₃O-Rest ist.

Die Substitution von Ar¹ und Ar² mit mindestens einem R-CO-Rest erfolgt vorzugsweise derart, dass der R-CO-Rest ein CH₃-CO-Rest ist.

In einer besonders bevorzugten Ausführungsform ist sowohl Ar¹ als auch Ar² ein Phenyl-Rest. Derartige Verbindungen sind relativ einfach und kostengünstig herzustellen und liefern hinsichtlich der nachstehend beschriebenen Eigenschaften gute Ergebnisse.

In einer bevorzugten Ausführungsform ist der Ar¹-SO₂-NH-Rest und der Ar²-NH-CO-NH-Rest in 4- bzw. 4' oder in 3- bzw. 3'-Stellung zu der -C₆H₄-SO₂-C₆H₄-Gruppe angeordnet. Besonders bevorzugt ist die Anordnung jeweils in 4- bzw. 4' Stellung, da derartige Verbindungen relativ einfach herstellbar sind und gute Eigenschaften zeigen. Anordnung in 4- bzw. 4'-Stellung bedeutet, dass der Ar¹-SO-NH- und der Ar²-NH-CO-NH-Rest jeweils in para-Stellung zur -C₄H₄-SO₂-C₆H₄-Gruppe angeordnet sind. Entsprechend bedeutet 3-bzw. 3'-Stellung eine Anordnung in meta-Stellung.

Der erfindungsgemäße Farbentwickler liegt vorzugsweise in einer Menge von etwa 3 bis etwa 35 Gew.-%, besonders bevorzugt in einer Menge von etwa 10 bis etwa 25 Gew.-%, bezogen auf den gesamten Feststoffgehalt der wärmeempfindlichen Schicht, vor.

Die Auswahl des Trägersubstrates ist nicht kritisch. Allerdings ist es bevorzugt, als Trägersubstrat Papier, synthetisches Papier und/oder eine Kunststoff-Folie einzusetzen.

Gegebenenfalls liegt zwischen dem Trägersubstrat und der wärmeempfindlichen Schicht mindestens eine weitere Zwischenschicht vor, wobei dieser Zwischenschicht die Aufgabe zukommt, die Oberflächenglätte des Trägers für die wärmeempfindliche Schicht zu verbessern und eine Wärmebarriere zwischen dem Trägerpapier und der wärmeempfindlichen Schicht zu realisieren; vorzugsweise kommen in dieser Zwischenschicht organische Hohlkugelpigmente und/oder kalzinierte Kaoline zum Einsatz.

Auch kann mindestens eine oberhalb der wärmeempfindlichen Schicht angeordnete Schutzschicht im erfindungsgemäßen wärmeempfindlichen Aufzeichnungsmaterial vorliegen. Des Weiteren kann das erfindungsgemäße wärmeempfindliche Aufzeichnungsmaterial mit einer Rückseitenpräparation ausgestattet sein. Dabei kann es sich im Einzelnen um eine Selbstklebeschicht oder um eine sog. "back-coat"-Schicht zur Verbesserung der rückseitigen Bedruckbarkeit oder zur Minimierung der Rollneigung ("curling") des wärmeempfindlichen Aufzeichnungsmaterial bei ungünstigen Feuchtebedingungen handeln. Auch rückseitig aufgebrachte magnetische/magnetisierbare Beschichtungen sind im Umfang der hier beschriebenen Rückseitenpräparationen enthalten.

Hinsichtlich der Wahl des Farbbildners unterliegt die vorliegende Erfindung ebenfalls keinen wesentlichen Einschränkungen. Bevorzugt ist der Farbbildner jedoch ein Farbstoff vom Triphenylmethan-, Fluoran-, Azaphthalid- und/oder Fluoren-Typ. Ein ganz besonders bevorzugter Farbbildner ist ein Farbstoff vom Fluoran-Typ, da dieser dank der Verfügbarkeit und der ausgewogenen anwendungsbezogenen Eigenschaften die Bereitstellung eines Aufzeichnungsmaterials mit einem attraktiven Preis-Leistungsverhältnis ermöglicht.

Besonders bevorzugte Farbstoffe vom Fluoran-Typ sind:
3-Diethylamino-6-methyl-7-anilinofluoran,
3-(*N*-Ethyl-*N*-4-toludinamino)-6-methyl-7-anilinofluoran,
3-(*N*-Ethyl-*N*-isoamylamino)-6-methyl-7-anilinofluoran,
3-Diethylamino-6-methyl-7-(2,4-dimethylanilino)fluoran,
3-Pyrrolidino-6-methyl-7-anilinofluoran,
3-(Cyclohexyl-*N*-methylamino)-6-methyl-7-anilinofluoran,
3-Diethylamino-7-(3-trifluoromethylanilino)fluoran,
3-*N*-n-Dibutylamino-6-methyl-7-anilinofluoran,
3-Diethylamino-6-methyl-7-(3-methylanilino)fluoran,
3-*N*-n-Dibutylamino-7-(2-chloranilino)fluoran,
3-(*N*-Ethyl-*N*-tetrahydrofurfurylamino)-6-methyl-7-anilinofluoran,
3-(*N*-Methyl-*N*-propylamino)-6-methyl-7-anilinofluoran,
3-(*N*-Ethyl-*N*-ethoxypropylamino)-6-methyl-7-anilinofluoran,
3-(*N*-Ethyl-*N*-isobutylamino)-6-methyl-7-anilinofluoran und/oder
3-Dipentylamino-6-methyl-7-anilinofluoran.

Die Farbbildner können als Einzelstoffe als auch als beliebige Gemische zweier oder mehrerer Farbbildner zur Anwendung kommen, vorausgesetzt, die wünschenswerten anwendungstechnischen Eigenschaften der Aufzeichnungsmaterialien leiden darunter nicht.

Der Farbbildner liegt vorzugsweise in einer Menge von etwa 5 bis etwa 30 Gew.- %, besonders bevorzugt in einer Menge von etwa 8 bis etwa 20 Gew.-%, bezogen auf den gesamten Feststoffgehalt der wärmeempfindlichen Schicht, vor.

Zur Steuerung spezieller anwendungstechnischer Eigenschaften kann es vorteilhaft sein, wenn ein oder mehrere weitere (bis)phenolische oder nichtphenolische Farbentwickler zusätzlich zu den bisher offenbarten Anwendungsformen des Farbentwicklers in der wärmeempfindlichen farbbildenden Schicht vorliegen.

Neben dem mindestens einen Farbbildner und dem mindestens einen Farbentwickler können in der wärmeempfindlichen farbbildenden Schicht ein oder mehrere Sensibilisierungsmittel, auch thermische Lösungsmittel oder Schmelzhilfsmittel genannt, vorliegen, was den Vorteil hat, dass die Steuerung der thermischen Druckempfindlichkeit leichter zu realisieren ist.

Generell kommen als Sensibilisierungsmittel vorteilhafterweise kristalline Stoffe in Betracht, deren Schmelzpunkt zwischen etwa 90 und etwa 150 °C liegt und die im geschmolzenen Zustand die farbbildenden Komponenten (Farbbildner und Farbentwickler) lösen, ohne die Ausbildung des Farbkomplexes zu stören.

Vorzugsweise ist das Sensibilisierungsmittel ein Fettsäureamid, wie Stearamid, Beheneamid oder Palmitamid, ein Ethylen-bis-Fettsäureamid, wie *N*,*N'*-Ethylen-bis-stearinsäureamid oder *N*,*N'*-Ethylen-bis-ölsäureamid, ein Fettsäurealkanolamid, wie *N*-(Hydroxymethyl)stearamid, *N-*Hydroxymethylpalmitamid oder Hydroxyethylstearamid, ein Wachs, wie Polyethylenwachs oder Montanwachs, ein Carbonsäureester, wie Dimethylterephthalat, Dibenzylterephthalat, Benzyl-4-benzyloxybenzoat, Di-(4-methylbenzyl)oxalat, Di-(4-chlorbenzyl)oxalat oder Di-(4-benzyl)oxalat, Ketone, wie 4-Acetylbiphenyl, ein aromatischer Ether, wie 1,2-Diphenoxy-ethan, 1,2-Di-(3-methylphenoxy)ethan, 2-Benzyloxynaphthalin, 1,2-Bis-(phenoxymethyl)benzol oder 1,4-Diethoxynaphthalin, ein aromatisches Sulfon, wie Diphenylsulfon, und/oder ein aromatisches Sulfonamid, wie 4-Toluolsulfonamid, Benzolsulfonanilid oder *N*-Benzyl-4-toluolsulfonamid oder aromatische Kohlenwasserstoffe, wie 4-Benzylbiphenyl.

In einer weiteren bevorzugten Ausführungsform liegt neben dem Farbbildner, dem phenolfreien Farbentwickler und dem Sensibilisierungsmittel optional mindestens ein Stabilisator (Alterungsschutzmittel) in der wärmeempfindlichen, farbbildenden Schicht vor.

Bei dem Stabilisator handelt es sich vorzugsweise um sterisch gehinderte Phenole, besonders bevorzugt um 1,1,3-Tris-(2-methyl-4-hydroxy-5-cyclohexyl-phenyl)-butan, 1,1,3-Tris-(2-methyl-4-hydroxy-5-tert-butylphenyl)-butan, 1,1-Bis-(2-methyl-4-hydroxy-5-tert-butyl-phenyl)-butan.

Auch Harnstoff-Urethan-Verbindungen (Handelsprodukt UU) oder vom 4,4'-Dihydroxydiphenylsulfon abgeleitete Ether, wie 4-Benzyloxy-4'-(2-methylglycidyloxy)-diphenylsulfon (Handelsname NTZ-95^{®}, Nippon Soda Co. Ltd.), oder oligomere Ether (Handelsname D90^{®}, Nippon Soda Co. Ltd.) sind als Stabilisatoren im erfindungsgemäßen Aufzeichnungsmaterial einsetzbar.

Der Stabilisator liegt vorzugsweise in einer Menge von 0,2 bis 0,5 Gew.-Teilen, bezogen auf den mindestens einen phenolfreien Farbentwickler der Verbindung der Formel (I), vor.

In einer weiteren bevorzugten Ausführungsform liegt in der wärmeempfindlichen farbbildenden Schicht mindestens ein Bindemittel vor. Bei diesem handelt es sich vorzugsweise um wasserlösliche Stärken, Stärkederivate, stärkebasierte Biolatices vom EcoSphere^{®}-Typ, Methylcellulose, Hydroxyethylcellulose, Carboxymethylcellulosen, partiell oder vollständig verseifte Polyvinylalkohole, chemisch modifizierte Polyvinylalkohole oder StyrolmaleinsäureanhydridCopolymere, Styrolbutadien-Copolymere, Acrylamid-(Meth)acrylat-Copolymere, Acrylamid-Acrylat-Methacrylat-Terpolymere, Polyacrylate, Poly(meth)-acrylsäureester, Acrylat-Butadien-Copolymere, Polyvinylacetate und/oder Acrylnitril-Butadien-Copolymere.

In einer weiteren bevorzugten Ausführungsform liegt mindestens ein Trennmittel (Antihaftmittel) oder Gleitmittel in der wärmeempfindlichen farbbildenden Schicht vor. Bei diesen Mitteln handelt es sich vorzugsweise um Fettsäure-Metallsalze, wie z. B. Zinkstearat oder Calciumstearat, oder auch Behenatsalze, synthetische Wachse, z. B. in Form von Fettsäureamiden, wie z. B. Stearinsäureamid und Behensäureamid, Fettsäurealkanolamide, wie z. B. Stearinsäure-methylolamid, Paraffinwachse verschiedener Schmelzpunkte, Esterwachse unterschiedlicher Molekulargewichte, Ethylenwachse, Propylenwachse unterschiedlicher Härten und/oder natürliche Wachse, wie z. B. Carnaubawachs oder Montanwachs.

In einer weiteren bevorzugten Ausführungsform enthält die wärmeempfindliche farbbildende Schicht Pigmente. Der Einsatz dieser hat unter anderem den Vorteil, dass diese auf ihrer Oberfläche die im thermischen Druckprozess entstehende Chemikalien-Schmelze fixieren können. Auch kann über Pigmente die Oberflächenweiße und Opazität der wärmeempfindlichen farbbildenden Schicht und deren Bedruckbarkeit mit konventionellen Druckfarben gesteuert werden. Schließlich besitzen Pigmente eine "Extenderfunktion", beispielsweise für die relativ teuren farbgebenden Funktionschemikalien.

Besonders geeignete Pigmente sind anorganische Pigmente, sowohl synthetischer als auch natürlicher Herkunft, vorzugsweise Clays, gefällte oder natürliche Calciumcarbonate, Aluminiumoxide, Aluminiumhydroxide, Kieselsäuren, gefällte und pyrogene Kieselsäuren (z. B. Aerodisp^{®}- Typen), Diathomeenerden, Magnesiumcarbonate, Talk, aber auch organische Pigmente, wie Hohlpigmente mit einer Styrol/Acrylat-Copolymer-Wand oder Harnstoff/Formaldehyd-Kondensationspolymere. Diese können alleine oder in beliebigen Mischungen verwendet werden.

Zum Steuern der Oberflächenweiße des erfindungsgemäßen wärmeempfindlichen Aufzeichnungsmaterials können optische Aufheller in die wärmeempfindliche farbbildende Schicht eingebaut werden. Bei diesen handelt es sich vorzugsweise um Stilbene.

Um bestimmte streichtechnische Eigenschaften zu verbessern, ist es im Einzelfall bevorzugt, zu den zwingenden Bestandteilen des erfindungsgemäßen wärmeempfindlichen Aufzeichnungsmaterials weitere Bestandteile, insbesondere Rheologie-Hilfsmittel, wie z. B. Verdicker und/oder Tenside, hinzuzufügen.

Das Flächenauftragsgewicht der (trockenen) wärmeempfindlichen Schicht beträgt vorzugsweise etwa 1 bis etwa 10 g/m², bevorzugt etwa 3 bis etwa 6 g/m².

In einer besonders bevorzugten Ausführungsform handelt es sich bei dem wärmeempfindlichen Aufzeichnungsmaterial um ein solches nach Anspruch 8, wobei als Farbbildner ein Farbstoff vom Fluoran-Typ eingesetzt wird und zusätzlich ein Sensibilisierungsmittel, ausgewählt aus der Gruppe bestehend aus Fettsäureamiden, aromatischen Sulfonen, Benzyloxalate und/oder aromatischen Ethern, vorliegt. In dieser bevorzugten Ausführungsform ist es auch vorteilhaft, dass etwa 1,5 bis etwa 4 Gew.-Teile des phenolfreien Farbentwicklers nach Anspruch 1, bezogen auf den Farbbildner, vorliegen.

Das erfindungsgemäße wärmeempfindliche Aufzeichnungsmaterial lässt sich mit bekannten Herstellungsverfahren gewinnen.

Es ist jedoch bevorzugt, das erfindungsgemäße Aufzeichnungsmaterial mit einem Verfahren zu gewinnen, bei dem auf ein Trägersubstrat eine die Ausgangsmaterialien der wärmeempfindlichen farbbildenden Schicht enthaltende wässrige Suspension aufgetragen und getrocknet wird, wobei die wässrige Auftragssuspension einen Feststoffgehalt von etwa 20 bis etwa 75 Gew.-%, bevorzugt von etwa 30 bis etwa 50 Gew.%, aufweist, und mit dem Curtain-Coating-Beschichtungsverfahren bei einer Betriebsgeschwindigkeit der Streichanlage von mindestens etwa 400 m/min aufgetragen und getrocknet wird.

Dieses Verfahren ist insbesondere unter wirtschaftlichen Gesichtspunkten vorteilhaft.

Wird der Wert des Feststoffgehaltes von etwa 20 Gew.-% unterschritten, dann verschlechtert sich die Wirtschaftlichkeit, da eine große Menge von Wasser aus dem Strich durch schonende Trocknung in kurzer Zeit entfernt werden muss, was sich nachteilig auf die Streichgeschwindigkeit auswirkt. Wird auf der anderen Seite der Wert von 75 Gew.-% überschritten, dann führt dies lediglich zu einem erhöhten technischen Aufwand, um die Stabilität des Streichfarben-Vorhangs während des Beschichtungsprozesses zu gewährleisten.

Beim Curtain-Coating-Beschichtungsverfahren (Vorhangbeschichtungsverfahren) wird ein frei fallender Vorhang einer Beschichtungsdispersion gebildet. Durch freien Fall wird die in Form eines dünnen Filmes (Vorhangs) vorliegende Beschichtungsdispersion auf ein Substrat "gegossen", um die Beschichtungsdispersion auf das Substrat aufzubringen. Die DE 10 196 052 T1 offenbart den Einsatz des Curtain-Coating-Beschichtungsverfahrens bei der Herstellung von Informationsaufzeichnungsmaterialien u.a. auch von wärmeempfindlichen Aufzeichnungsmaterialien, wobei mehrschichtige Aufzeichnungsschichten durch Aufbringen des aus mehreren Beschichtungsdispersionsfilmen bestehenden Vorhangs auf Substrate realisiert werden (Geschwindigkeit max. 200 m/min).

Die Einstellung der Betriebsgeschwindigkeit der Streichanlage auf mindestens etwa 400 m/min hat sowohl betriebswirtschaftliche als auch technische Vorteile. Bevorzugt beträgt die Betriebsgeschwindigkeit mindestens etwa 750 m/min, besonders bevorzugt mindestens etwa 1000 m/min und ganz besonders bevorzugt mindestens etwa 1500 m/min. Es war insbesondere überraschend, dass selbst bei letztgenannter Geschwindigkeit das erhaltene wärmeempfindliche Aufzeichnungsmaterial in keiner Weise beeinträchtigt ist und die Betriebsdurchführung selbst bei dieser hohen Geschwindigkeit optimal abläuft.

In einer bevorzugten Ausführungsform des erfindungsgemäßen Verfahrens weist die wässrige entlüftete Auftragssuspension eine Viskosität von etwa 150 bis etwa 800 mPas (Brookfield, 100 U/min, 20 °C) auf. Wird der Wert von etwa 150 mPas unterschritten bzw. der Wert von etwa 800 mPas überschritten, dann führt dies zu einer mangelhaften Lauffähigkeit der Streichmasse am Streichaggregat. Besonders bevorzugt beträgt die Viskosität der wässrigen entlüfteten Auftragssuspension etwa 200 bis etwa 500 mPas.

In einer bevorzugten Ausführungsform kann zur Optimierung des Verfahrens die Oberflächenspannung der wässrigen Auftragssuspension auf etwa 25 bis etwa 60 mN/m, bevorzugt auf etwa 35 bis etwa 50 mN/m (gemessen entsprechend der statischen Ringmethode nach Du Noüy, DIN 53914), eingestellt werden.

Die Ausbildung der wärmeempfindlichen farbbildenden Schicht kann on-line oder in einem separaten Streichvorgang off-line erfolgen. Dies gilt auch für eventuell nachfolgend aufgetragene Schichten oder Zwischenschichten.

Es ist vorteilhaft, wenn die getrocknete wärmeempfindliche farbbildende Schicht einer Glätt-Maßnahme unterzogen wird.

Hierbei ist es vorteilhaft, die Bekk-Glätte, gemessen nach ISO 5627:1995-03, auf etwa 100 bis etwa 1000 Sek., vorzugsweise auf etwa 250 bis etwa 600 Sek., einzustellen.

Die Oberflächenrauigkeit (PPS) nach ISO 8791-4:2008-05 liegt im Bereich von etwa 0,50 bis etwa 2,50 µm, vorzugsweise zwischen 1,00 und 2,00 µm.

Die Erfindung wird nachfolgend anhand nicht beschränkender Beispiele im Detail erläutert.

### Beispiel 1:

Die nicht phasenreine Verbindung *N*-(4-((4-(3-Phenylureido)phenyl)sulfonyl)phenyl)benzolsulfonamid wurde wie folgt hergestellt:
Zu einem Gemisch aus 7,5 mmol 4,4'-Diaminodiphenylsulfon und 7,5 mmol Pyridin in 80 mL Dichlormethan wird eine Lösung aus 7,5 mmol Phenylisocyanat in 20 ml Dichlormethan bei 0 °C unter Rühren tropfenweise gegeben. Die Reaktionslösung wird 16 Stunden bei Raumtemperatur gerührt. Anschließend wird eine Lösung aus 7,5 mmol Benzolsulfonylchlorid in 15 ml Dichlormethan bei 0 °C unter Rühren tropfenweise gegeben. Das Reaktionsgemisch wird refluxiert und der Reaktionsverlauf mittels HPLC verfolgt. Nach Beenden der Reaktion wird das Produkt abfiltriert, mit Dichlormethan gewaschen und im Vakuum getrocknet.

### Beispiel 2:

6,84 g *N*-(4-((4-(3-Phenylureido)phenyl)sulfonyl)phenyl)benzolsulfonamid, hergestellt gemäß Beispiel 1, wird aus EtOAc/n-Hexan umkristallisiert. Erhalten wird *N*-(4-((4-(3-Phenylureido)phenyl)sulfonyl)phenyl)benzolsulfonamid, charakterisiert durch ein Röntgenpulverdiffraktogramm mit folgenden Braggschen Winkeln (2θ/CuK_{α}): 8,7, 9,8, 10,8, 13,2, 13,9, 14,9, 15,2, 16,0, 17,4, 17,7, 18,7, 20,4, 21,2, 21,6, 22,3, 23,0, 23,3, 23,9, 24,4, 25,0, 25,8, 26,6, 28,1, 28,9, 29,4, 30,1, 30,6, 31,8, 34,5, 35,3, 35,6, 36,9 (s. Fig. 2). Diese Verbindung wird in 225 ml Ethylacetat suspendiert und für sechs Stunden refluxiert. Nach dem Abkühlen wird das Lösungsmittel entfernt und *N*-(4-((4-(3-Phenylureido)phenyl)sulfonyl)phenyl)benzolsulfonamid, charakterisiert durch ein Röntgenpulverdiffraktogramm mit folgenden Braggschen Winkeln (2θ/CuK_{α}): 10,9, 11,7, 14,6, 15,0, 15,8, 16,6, 17,6, 18,9, 19,4, 20,9, 21,2, 22,0, 23,3, 24,4, 24,7, 26,1, 27,4, 29,4, 34,2 (s. Fig. 1), erhalten.

### Beispiel 3:

*N*-(4-((4-(3-Phenylureido)phenyl)sulfonyl)phenyl)benzolsulfonamid, charakterisiert durch ein Röntgenpulverdiffraktogramm mit folgenden Braggschen Winkeln (2θ/CuK_{α}): 10,9, 11,7, 14,6, 15,0, 15,8, 16,6, 17,6, 18,9, 19,4, 20,9, 21,2, 22,0, 23,3, 24,4, 24,7, 26,1, 27,4, 29,4, 34,2 (s. Fig. 1), wird aus Acetonitril umkristallisiert. Das erhaltene *N*-(4-((4-(3-Phenylureido)phenyl)sulfonyl)phenyl)benzolsulfonamid ist charakterisiert durch ein Röntgenpulverdiffraktogramm mit folgenden Braggschen Winkeln (2θ/CuK_{α}): 8,7, 9,8, 10,8, 13,2, 13,9, 14,9, 15,2, 16,0, 17,4, 17,7, 18,7, 20,4, 21,2, 21,6, 22,3, 23,0, 23,3, 23,9, 24,4, 25,0, 25,8, 26,6, 28,1, 28,9, 29,4, 30,1, 30,6, 31,8, 34,5, 35,3, 35,6, 36,9 (s. Fig. 2).

### Beispiel 4:

Es wurde ein wärmeempfindliches Aufzeichnungsmaterial bzw. Thermopapier hergestellt, wobei die folgenden Rezepturen wässriger Auftragssuspensionen zur Ausbildung eines Verbundgebildes auf einem Trägersubstrat herangezogen und anschließend in üblicher Weise die weiteren Schichten, insbesondere eine Schutzschicht, ausgebildet wurden, worauf hier nicht gesondert eingegangen werden soll.

Der Auftrag einer wässrigen Auftragssuspension zur Ausbildung der wärmeempfindlichen farbbildenden Schicht eines wärmeempfindlichen Aufzeichnungspapiers erfolgte im Labormaßstab mittels einer Stabrakel auf eine Seite eines synthetischen Basispapieres (Yupo^{®} FP680) von 63 g/m². Nach Trocknung wurde ein thermisches Aufzeichnungsblatt erhalten. Die Auftragsmenge der wärmeempfindlichen farbbildenden Schicht lag zwischen 3,8 und 4,2 g/m².

Herstellen der Dispersionen (jeweils für 1 Gew.-Teil) für die Auftragssuspensionen:
Die wässrige **Dispersion A** (Farbbildnerdispersion) wird durch Mahlen von 20 Gew.-Teilen 3-*N*-n-Dibutylamin-6-methyl-7-anilinofluoran (ODB-2) mit 33 Gew.-Teilen einer 15%igen wässrigen Lösung von Ghosenex^{™} L-3266 (sulfonierter Polyvinylalkohol, Nippon Ghosei) in einer Perlen-Mühle hergestellt.

Die wässrige **Dispersion B** (Farbentwicklerdispersion) wird durch Mahlen von 40Gew.-Teilen des jeweiligen Farbentwicklers (*N*-(4-((4-(3-Phenylureido)phenyl)sulfonyl)phenyl)benzolsulfonamid, Form ω^{18,9} bzw. Form α^{21,2} zusammen mit 66 Gew.-Teilen einer 15%igen wässrigen Lösung von Ghosenex^{™} L-3266 in einer Perlen-Mühle hergestellt.

Die wässrige **Dispersion C** (Sensibilisierungsdispersion) wird durch Mahlen von 40 Gew.-Teilen des jeweiligen Sensitizers mit 33 Gew.-Teilen einer 15%igen wässrigen Lösung von Ghosenex^{™} L-3266 in einer Perlen-Mühle hergestellt.

Es kamen folgende Sensibilisierungsmittel zur Anwendung: 1,2-Diphenoxyethan (DPE), Stearamid (SA), Diphenylsulfon (DPS), Di-(4-methylbenzyl)oxalat (HS3520), Benzyloxynaphthalin (BON), 1,2-Di(3-methylphenoxy)ethan (EGTE).

Alle durch Mahlen erzeugten Dispersionen haben eine mittlere Körngröße D**_{(4,3)}** von 0,80 bis 1,20 µm. Die Messung der Korngrößenverteilung der Dispersionen erfolgte durch Laserbeugung mit einem Coulter LS230-Gerät der Fa. Beckman Coulter.

Die **Dispersion D** (Gleitmitteldispersion) ist eine 20%ige Zinkstearat-Dispersion, bestehend aus 9 Gew.-Teilen Zn-Stearat, 1 Gew.-Teil Ghosenex^{™} L-3266 und 40 Gew.-Teilen Wasser.

Pigment **P** ist eine 72%ige Streichkaolin-Suspension (Lustra^{®} S, BASF).

Der **Binder** besteht aus einer 10%igen wässrigen Polyvinylalkohollösung (Mowiol 28-99, Kuraray Europe).

Die wärmeempfindliche Auftragssuspension wird durch Mischen unter Rühren von 1 Teil **A**, 1 Teil **B,** 1 Teil **C,** 56 Teilen **D,** 146 Teilen Pigment **P** und 138 Teilen **Binder**-Lösung (alles Gew.-Teile) unter Berücksichtigung der Eintragsreihenfolge **B, D, C, P, A, Binder** hergestellt und mit Wasser auf einen Feststoffgehalt von etwa 25% gebracht.

Die so erhaltenen wärmeempfindlichen Beschichtungssuspensionen wurden herangezogen, um Verbundgebilde aus Papierträger und Thermoreaktionsschicht herzustellen.

Die thermischen Aufzeichnungsmaterialien wurden wie nachstehend beschrieben ausgewertet (siehe Tabelle 2).
(1) Dynamische Farbdichte:
   Die Papiere (6 cm breite Streifen) wurden thermisch unter Verwendung des Atlantek 200 Testdruckers (Fa. Atlantek, USA) mit einer Kyocera-Druckleiste von 200 dpi und 560 Ohm bei einer angelegten Spannung von 20,6 V und einer maximalen Pulsbreite von 0,8 ms mit einem Schachbrett-Muster mit 10 Energieabstufungen bedruckt. Die Bilddichte (optische Dichte, o.D.) wurde mit einem SpectroEye-Densitometer von X-Rite bei einer Energiestufe von 0,45 mJ/dot gemessen. Für die Auswertung wurde der jeweils höchste o.D.-Wert des Schachbrett-Musters berücksichtigt (Tabelle 2). Die Messunsicherheit der o.D.-Werte wird mit ≤2%veranschlagt.
(2) Statische Farbdichte (Starttemperatur):
   Das Aufzeichnungsblatt wurde gegen eine Reihe auf unterschiedliche Temperaturen erhitzter und thermostatierter metallischer Stempel mit einem Anpressdruck von 0,2 kg/cm² und einer Kontaktzeit von 5 Sek. Gepresst (Thermoprüfer TP 3000QM, Maschinenfabrik Hans Rychiger AG, Steffisburg, Schweiz). Die Bilddichte (optische Dichte) der so erzeugten Bilder wurde mit einem SpectroEye-Densitometer von X-Rite gemessen.

Der statische Startpunkt ist definitionsmäßig die niedrigste Temperatur in °C, bei welcher eine optische Dichte von 0,2 erreicht wird. Die Genauigkeit des Messverfahrens ist ≤±0,5 °C.

Die Tabelle 2 fasst die Auswertung der gefertigten Aufzeichnungsmaterialien zusammen.

**Tabelle 2**

| Polymorphe Form des FE | Sensibilisierungsmittel | o. D. max. | Startpunkt (°C) |
|---|---|---|---|
| ω^{18,9} | EGTE | 1,29 | 93 |
| α^{21,2} | | 1,27 | 91 |
| ω^{18,9} | DPE | 1,30 | 85 |
| α^{21,2} | | 1,29 | 84 |
| ω^{18,9} | SA | 1,19 | 92 |
| α^{21,2} | | 1,18 | 89 |
| ω^{18,9} | BON | 1,24 | 91 |
| α^{21,2} | | 1,24 | 88 |
| ω^{18,9} | DPS | 1,22 | 91 |
| α^{21,2} | | 1,22 | 87 |
| ω^{18,9} | HS 3520 | 1,28 | 90 |
| α^{21,2} | | 1,27 | 89 |

Den vorstehenden Beispielen lässt sich entnehmen, dass das wärmeempfindliche Aufzeichnungsmaterial der vorliegenden Erfindung insbesondere die folgenden vorteilhaften Eigenschaften zeigt:
(1) Sowohl der Einsatz der α^{21,2}-Form als auch der Einsatz der ω^{18,9}-Form als Farbentwickler führen zu hohen maximalen Druckdichten der erfindungsgemäßen wärmempfindlichen Aufzeichnungsmaterialien sowie zu hohen Temperaturen, bei der eine visuell merkliche Vergrauung der erfindungsgemäßen wärmempfindlichen Aufzeichnungsmaterialien (Startpunkt °C) eintritt.
(2) Die Temperatur, ab welcher eine visuell merkliche Vergrauung der erfindungsgemäßen Aufzeichnungsmaterialien eintritt (statischer Startpunkt), ist unter Verwendung des Polymorphs ω^{18,9} höher als bei den Beispielen mit der α^{21,2}-Form des Farbentwicklers. Daraus ergibt sich zum einen die Möglichkeit des Einsatzes der mit der ω^{18,9} Form gefertigten wärmeempfindlichen Aufzeichnungsmaterialien auch bei höheren Umgebungstemperaturen, zum anderen kommt eine thermisch weniger empfindliche Beschichtung der Prozessführung beim Trocknen der wässrigen Beschichtung zugute (Tabelle 2).
(3) Das aufgezeichnete Bild der mit der ω^{18,9}-Form des Farbentwicklers erhaltenen wärmeempfindlichen Aufzeichnungsmaterialien weist praktisch die gleiche hohe maximale Druckdichte (optische Dichte) auf, wie jenes der α^{21,2}-Form (Tabelle 2).
(4) Sowohl der Startpunkt als auch die dynamisch während des Druckprozessesgenerierte Druckdichte kann durch das Sensibilisierungsmittel in erheblichem Maße beeinflusst werden. Die Daten aus Tabelle 2 dokumentieren, dass die vorteilhaften Eigenschaften gemäß (2) und (3) für eine repräsentative Auswahl von Sensibilisierungsmittel Gültigkeit haben.

## Patentansprüche

1. *N*-(4-((4-(3-Phenylureido)phenyl)sulfonyl)phenyl)benzolsulfonamid in der polymorphen Form ω^{18,9}, charakterisiert durch ein Röntgenpulverdiffraktogramm mit den Braggschen Winkeln (2θ/CuK_{α}) 10,9, 11,7, 14,6, 15,0, 15,8, 16,6, 17,6, 18,9, 19,4, 20,9, 21,2, 22,0, 23,3, 24,4, 24,7, 26,1, 27,4, 29,4, 34,2,
oder in der polymorphen Form α^{21,2} charakterisiert durch ein Röntgenpulverdiffraktogramm mit den Braggschen Winkeln (2θ/CuK_{α}) 8,7, 9,8, 10,8, 13,2, 13,9, 14,9, 15,2, 16,0, 17,4, 17,7, 18,7, 20,4, 21,2, 21,6, 22,3, 23,0, 23,3, 23,9, 24,4, 25,0, 25,8, 26,6, 28,1, 28,9, 29,4, 30,1, 30,6, 31,8, 34,5, 35,3, 35,6, 36,9.

2. *N-*(4-((4-(3-Phenylureido)phenyl)sulfonyl)phenyl)benzolsulfonamid in der polymorphen Form ω^{18,9}oder in der polymorphen Form α^{21,2}gemäß Anspruch 1, **dadurch gekennzeichnet, dass** die polymorphe Form ω^{18,9}charakteristische Absorptionsbanden gemäß Fourier-Transformationsinfrarotspektroskopie bei den Wellenlängen 1092 cm⁻¹, 1105 cm⁻¹, 1145 cm⁻¹, 1233 cm⁻¹, 1319 cm⁻¹, 1495 cm⁻¹, 1548 cm⁻¹, 1598 cm⁻¹, 1655 cm⁻¹ und 3239 cm⁻¹ aufweist und
die polymorphe Form α^{21,2}charakteristische Absorptionsbanden gemäß Fourier-Transformationsinfrarotspektroskopie bei den Wellenlängen 1089 cm⁻¹, 1110 cm⁻¹, 1149 cm⁻¹, 1237 cm⁻¹, 1311 cm⁻¹, 1321 cm⁻¹, 1500 cm⁻¹, 1556 cm⁻¹, 1597 cm⁻¹, 1697 cm⁻¹, 3365 cm⁻¹ und 3408 cm⁻¹ aufweist.

3. *N-*(4-((4-(3-Phenylureido)phenyl)sulfonyl)phenyl)benzolsulfonamid in der polymorphen Form ω^{18,9}oder in der polymorphen Form α^{21,2} gemäß Anspruch 1 oder 2, **dadurch gekennzeichnet, dass** die polymorphe Form ω^{18,9} einen Schmelzbereich von 232 bis 233 °C und
die polymorphe Form α^{21,2} einen Schmelzbereich von 219 bis 221°C aufweist.

4. Verfahren zur Herstellung von *N*-(4-((4-(3-Phenylureido)phenyl)sulfonyl)phenyl)benzolsulfonamid in der polymorphen Form ω^{18,9}, **dadurch gekennzeichnet, dass** phasenreines *N*-(4-((4-(3-Phenylureido)phenyl)sulfonyl)phenyl)benzolsulfonamid in der polymorphen Form α^{21,2}, in einem organischen Lösungsmittel, vorzugsweise Ethylacetat, erhitzt, vorzugsweise refluxiert, wird.

5. Verfahren zur Herstellung von *N*-(4-((4-(3-Phenylureido)phenyl)sulfonyl)phenyl)benzolsulfonamid in der polymorphen Form α^{21,2}, **dadurch gekennzeichnet, dass** nicht phasenreines *N*-(4-((4-(3-Phenylureido)phenyl)sulfonyl)phenyl)benzolsulfonamid durch Umkristallisation aus einer Mischung von Ethylacetat und n-Hexan erhalten wird oder dass phasenreines *N*-(4-((4-(3-Phenylureido)phenyl)sulfonyl)phenyl)benzolsulfonamid in der polymorphen Form ω^{18,9}aus Acetonitril umkristallisiert wird.

6. *N-*(4-((4-(3-Phenylureido)phenyl)sulfonyl)phenyl)benzolsulfonamid in der polymorphen Form ω^{18,9}, erhältlich gemäß dem Verfahren nach Anspruch 4.

7. *N-*(4-((4-(3-Phenylureido)phenyl)sulfonyl)phenyl)benzolsulfonamid in der polymorphen Form α^{21,2},erhältlich gemäß dem Verfahren nach Anspruch 5.

8. Wärmeempfindliches Aufzeichnungsmaterial, umfassend ein Trägersubstrat sowie eine mindestens einen Farbbildner und mindestens einen phenolfreien Farbentwickler enthaltende wärmeempfindliche farbbildende Schicht, wobei der mindestens eine phenolfreie Farbentwickler *N*-(4-((4-(3-Phenylureido)phenyl)sulfonyl)phenyl)benzolsulfonamid in der polymorphen Form ω^{18,9} und/oder in der polymorphen Form α^{21,2}, gemäß irgendeinem der Ansprüche 1 bis 3, 6 oder 7 ist.

9. Wärmeempfindliches Aufzeichnungsmaterial nach Anspruch 8, wobei der Farbentwickler in einer Menge von etwa 3 bis etwa 35 Gew.-%, bevorzugt von etwa 10 bis etwa 25 Gew.-%, bezogen auf den gesamten Feststoffgehalt der wärmeempfindlichen Schicht, vorliegt.

10. Wärmeempfindliches Aufzeichnungsmaterial nach Anspruch 8 bis 9, wobei der mindestens eine Farbbildner ein Farbstoff vom Triphenylmethan-Typ, vom Fluoran-Typ, vom Azaphthalid-Typ und/oder vom Fluoren-Typ, bevorzugt vom Fluoran-Typ, ist.

11. Wärmeempfindliches Aufzeichnungsmaterial nach mindestens einem der Ansprüche 8 bis 10, wobei neben dem phenolfreien Farbentwickler mindestens ein Farbentwickler der allgemeinen Formel
Ar¹-SO₂-NH-C₆H₄-SO₂-C₆H₄-NH-CO-NH-Ar²,
wobei Ar¹ und Ar² ein unsubstituierter oder substituierter Phenyl-Rest ist, vorliegt.

12. Wärmeempfindliches Aufzeichnungsmaterial nach Anspruch 11, **dadurch gekennzeichnet, dass** Ar¹ ein Phenyl-Rest ist und /oder dass Ar² ein Phenyl-Rest ist.

13. Wärmeempfindliches Aufzeichnungsmaterial nach Anspruch 11 oder 12, **dadurch gekennzeichnet, dass** Ar¹ mit mindestens einem C₁-C₅-Alkyl-, einem Alkenyl-, einem Alkinyl-, einem Benzyl-, einem Formyl-, einem CN-, einem Halogen-, einem NO₂-, einem RO-, einem R-CO-, einem RO₂C-, einem R-OCO-, einem R-SO₂O-, einem R-O-SO₂-, einem R-SO₂-NH-, einem R-NH-SO₂-, einem R-NH-CO- oder einem R-CO-NH-Rest, wobei R ein C₁-C₅-Alkyl-, ein Alkenyl-, ein Alkinyl-, ein Phenyl-, ein Tolyl- oder ein Benzyl-Rest ist, substituiert ist.

14. Verfahren zur Herstellung eines wärmeempfindlichen Aufzeichnungsmaterials nach mindestens einem der Ansprüche 8 bis 13, wobei auf ein Trägersubstrat eine die Ausgangsmaterialien der wärmeempfindlichen farbbildenden Schicht enthaltende wässrige Suspension aufgetragen und getrocknet wird, wobei die wässrige Auftragssuspension einen Feststoffgehalt von etwa 20 bis etwa 75 Gew.-%, bevorzugt von etwa 30 bis etwa 50 Gew.%, aufweist, und mit dem Curtain-Coating-Beschichtungsverfahren bei einer Betriebsgeschwindigkeit der Streichanlage von mindestens etwa 400 m/min, bevorzugt von mindestens etwa 1000 m/min, ganz besonders bevorzugt von mindestens etwa 1500 m/min, aufgetragen und getrocknet wird.

15. Wärmeempfindliches Aufzeichnungsmaterial, erhältlich gemäß dem Verfahren nach Anspruch 14.

## Claims

1. An *N*-(4-((4-(3-phenylureido)phenyl)sulfonyl)phenyl)benzenesulfonamide in the polymorphic form ω^{18.9}, **characterised by** an x-ray powder diffractogram having the Bragg angles (2θ/CuK_{α}) 10.9, 11.7, 14.6, 15.0, 15.8, 16.6, 17.6, 18.9, 19.4, 20.9, 21.2, 22.0, 23.3, 24.4, 24.7, 26.1, 27.4, 29.4, 34.2, or in the polymorphic form α^{21.2}, **characterised by** an x-ray powder diffractogram having the Bragg angles (2θ/CuK_{α}) 8.7, 9.8, 10.8, 13.2, 13.9, 14.9, 15.2, 16.0, 17.4, 17.7, 18.7, 20.4, 21.2, 21.6, 22.3, 23.0, 23.3, 23.9, 24.4, 25.0, 25.8, 26.6, 28.1, 28.9, 29.4, 30.1, 30.6, 31.8, 34.5, 35.3, 35.6, 36.9.

2. The *N*-(4-((4-(3-phenylureido)phenyl)sulfonyl)phenyl)benzenesulfonamide in the polymorphic form ω^{18.9} or in the polymorphic form α^{21.2} according to claim 1, **characterised in that** the polymorphic form ω^{18.9} has characteristic absorption bands according to Fourier transformation infrared spectroscopy at the wavelengths 1092 cm⁻¹, 1105 cm⁻¹,1145 cm⁻¹, 1233 cm⁻¹, 1319 cm⁻¹, 1495 cm⁻¹, 1548 cm⁻¹, 1598 cm⁻¹, 1655 cm⁻¹ and 3239 cm⁻¹ and the polymorphic form a α^{21.2} has characteristic absorption bands according to Fourier transformation infrared spectroscopy at the wavelengths 1089 cm⁻¹, 1110 cm⁻¹, 1149 cm⁻¹, 1237 cm⁻¹, 1311 cm⁻¹, 1321 cm⁻¹, 1500 cm⁻¹, 1556 cm⁻¹, 1597 cm⁻¹, 1697 cm⁻¹, 3365 cm⁻¹ and 3408 cm⁻¹.

3. The *N*-(4-((4-(3-phenylureido)phenyl)sulfonyl)phenyl)benzenesulfonamide in the polymorphic form ω^{18.9} or in the polymorphic form α^{21.2} according to claim 1 or 2, **characterised in that** the polymorphic form ω^{18.9} has a melting range of 232 to 233 °C and the polymorphic form α^{21.2} has a melting range of 219 to 221 °C.

4. A method for producing *N*-(4-((4-(3-phenylureido)phenyl)sulfonyl)phenyl)benzenesulfonamide in the polymorphic form ω^{18.9}, **characterised in that** monophase *N*-(4-((4-(3-phenylureido)phenyl)sulfonyl)phenyl)benzenesulfonamide in the polymorphic form α^{21.2} is heated, preferably refluxed, in an organic solvent, preferably ethyl acetate.

5. A method for producing *N*-(4-((4-(3-phenylureido)phenyl)sulfonyl)phenyl) benzenesulfonamide in the polymorphous form α^{21.2}, **characterised in that** non-monophase *N*-(4-((4-(3-phenylureido)phenyl)sulfonyl)phenyl)benzenesulfonamide is obtained by recrystallisation from a mixture of ethyl acetate and n-hexane, or **in that** monophase *N*-(4-((4-(3-phenylureido)phenypsulfonyl)phenyl)benzenesulfonamide in the polymorphic form ω^{18.9} is recrystallised from acetonitrile.

6. An *N*-(4-((4-(3-phenylureido)phenyl)sulfonyl)phenyl)benzenesulfonamide in the polymorphic form ω^{18.9}, obtained by the method according to claim 4.

7. An *N*-(4-((4-(3-phenylureido)phenyl)sulfonyl)phenyl)benzenesulfonamide in the polymorphic form α^{21.2}, obtained by the method according to claim 5.

8. A heat-sensitive recording material, comprising a carrier substrate and a heat-sensitive colour-forming layer containing at least one colour former and at least one phenol-free colour developer, wherein the at least one phenol-free colour developer is *N*-(4-((4-(3-phenylureido)phenyl)sulfonyl)phenyl)benzenesulfonamide in the polymorphic form ω^{18.9} and/or in the polymorphic form α^{21.2}, according to any one of the claims 1 to 3, 6 or 7.

9. The heat-sensitive recording material according to claim 8, wherein the colour developer is present in an amount of from about 3 to about 35%, preferably of from about 10 to 25% by weight in relation to the total solids content of the heat-sensitive layer.

10. The heat-sensitive recording material according to any one of claims 8 or 9, wherein the at least one colour former is a dye of the triphenylmethane type, of the fluoran type, of the azaphthalide type and/or of the fluorene type, preferably the fluoran type.

11. The heat-sensitive recording material according to any one of claims 8 to 10, wherein, besides the phenol-free colour developer, at least one colour developer of general formula
Ar¹-SO₂-NH-C₆H₄-SO₂-C₆H₄-NH-CO-NH-Ar²,
wherein Ar¹ and Ar² are an unsubstituted or substituted phenyl group, is also present.

12. The heat-sensitive recording material according to claim 11, **characterised in that** Ar¹ is a phenyl group, and/or **in that** Ar² is a phenyl group.

13. The heat-sensitive recording material according to any one of claims 11 or 12, **characterised in that** Ar¹ is substituted with at least one C₁-C₅ alkyl group, an alkenyl group, an alkynyl group, a benzyl group, a formyl group, a CN group, a halogen group, an NO₂ group, an RO group, an R-CO group, an RO₂C group, an R-OCO group, an R-SO₂O group, an R-O-SO₂ group, an R-SO₂-NH group, an R-NH-SO₂ group, an R-NH-CO group or an R-CO-NH group, wherein R is a C₁-C₅ alkyl group, an alkenyl group, an alkynyl group, a phenyl group, a tolyl group, or a benzyl group.

14. A method for producing a heat-sensitive recording material according to any one of claims 8 to 13, wherein an aqueous suspension containing the starting materials of the heat-sensitive colour-forming layer is applied to a carrier substrate and dried, wherein the aqueous application suspension has a solids content of from about 20 to about 75% by weight, preferably of from about 30 to about 50% and is applied and dried by the curtain coating process at an operating speed of the coating plant of at least about 400 m/min, preferably of at least about 1000 m/min, more preferably of at least about 1500 m/min.

15. Heat-sensitive recording material obtained by the process according to claim 14.

## Revendications

1. N-(4-((4-(3-phényluréido)phényle)sulfonyle)phényle)ben zolsulfonamide sous la forme polymorphe ω^{18,9}, **caractérisée par** un diffractogramme de poudre de rayons x ayant les angles de Bragg (2θ/CuKα) 10,9, 11,7, 14,6, 15,0, 15,8, 16,6, 17,6, 18,9, 19,4, 20,9, 21,2, 22,0, 23,3, 24,4, 24,7, 26,1, 27,4, 29,4, 34,2
ou sous la forme polymorphe α21,2 **caractérisée par** un diffractogramme de poudre de rayons x ayant les angles de Bragg (2θ/CuKα) 8,7, 9,8, 10,8, 13,2, 13,9, 14,9, 15,2, 16,0, 17,4, 17,7, 18,7, 20,4, 21,2, 21,6, 22,3, 23,0, 23,3, 23,9, 24,4, 25,0, 25,8, 26,6, 28,1, 28,9, 29,4, 30,1, 30,6, 31,8, 34,5, 35,3, 35,6, 36,9.

2. N-(4-((4-(3-phényluréido)phényle)sulfonyle)phényle)ben zolsulfonamide sous la forme polymorphe ω^{18,9} ou sous la forme polymorphe α^{21,2} selon la revendication 1, **caractérisé en ce que** la forme polymorphe ω^{18,9} présente des bandes d'absorption caractéristiques selon la spectroscopie infrarouge par transformation de Fourier pour les longueurs d'onde 1092 cm⁻¹, 1105 cm⁻¹, 1145 cm⁻¹, 1233 cm⁻¹, 1319 cm⁻¹, 1495 cm⁻¹, 1548 cm⁻¹, 1598 cm⁻¹, 1655 cm⁻¹ et 3239 cm⁻¹ et
la forme polymorphe α^{21,2} présente des bandes d'absorption caractéristiques selon la spectroscopie infrarouge par transformation de Fourier pour les longueurs d'onde 1089 cm⁻¹, 1110 cm⁻¹, 1149 cm⁻¹, 1237 cm⁻¹, 1311 cm⁻¹, 1321 cm⁻¹, 1500 cm⁻¹, 1556 cm⁻¹, 1597 cm⁻¹, 1697 cm⁻¹, 3365 cm⁻¹ et 3408 cm⁻¹.

3. N-(4-((4-(3-phényluréido)phényle)sulfonyle)phényle)ben zolsulfonamide sous la forme polymorphe ω^{18,9} ou sous la forme polymorphe α^{21,2} selon la revendication 1 ou 2, **caractérisé en ce que** la forme polymorphe w^{18,9}présente une plage de fusion de 232 à 233 °C et la forme polymorphe α^{21,2} présente une plage de fusion de 219 à 221 °C.

4. Procédé de fabrication de N-(4-((4-(3-phényluréido)phényle)sulfonyle)phényle)benzol sulfonamide sous la forme polymorphe ω^{18,9}, **caractérisé en ce que** du N-(4-((4-(3-phényluréido)phényle)sulfonyle)phényle)benzol sulfonamide sous la forme polymorphe α^{21,2} à phases pures est chauffé, de préférence est chauffé au reflux, dans un solvant organique, de préférence dans de l'acétate d'éthyle.

5. Procédé de fabrication de N-(4-((4-(3-phényluréido)phényle)sulfonyle)phényle)benzol sulfonamide sous la forme polymorphe α^{21,2}, **caractérisé en ce que** du N-(4-((4-(3-phényluréido)phényle)sulfonyle)phényle)benzol sulfonamide sans phases pures est obtenu par recristallisation à partir d'un mélange d'acétate d'éthyle et de n-hexane, ou que du N-(4-((4-(3-phényluréido)phényle)sulfonyle)phényle)benzol sulfonamide sous la forme polymorphe ω^{18,9} à phases pures est recristallisé à partir d'acétonitrile.

6. N-(4-((4-(3-phényluréido)phényle)sulfonyle)phényle)ben zolsulfonamide sous la forme polymorphe ω^{18,9} pouvant être obtenu selon le procédé selon la revendication 4.

7. N-(4-((4-(3-phényluréido)phényle)sulfonyle)phényle)ben zolsulfonamide sous la forme polymorphe α^{21,2} pouvant être obtenu selon le procédé selon la revendication 5.

8. Matériau d'enregistrement thermosensible comprenant un substrat de support ainsi qu'une couche chromogène thermosensible contenant au moins un agent chromogène et au moins un révélateur chromogène non phénolique, dans lequel l'au moins un révélateur chromogène non phénolique est du N-(4-((4-(3-phényluréido)phényle)sulfonyle)phényle)benzol sulfonamide sous la forme polymorphe ω^{18,9} et/ou sous la forme polymorphe α^{21,2} selon l'une quelconque des revendications 1 à 3, 6 ou 7.

9. Matériau d'enregistrement thermosensible selon la revendication 8, dans lequel le révélateur chromogène est présent en une quantité d'environ 3 à environ 35 % en poids, de manière préférée d'environ 10 à environ 25 % en poids, par rapport à la teneur totale en matières sèches de la couche thermosensible.

10. Matériau d'enregistrement thermosensible selon la revendication 8 à 9, dans lequel l'au moins un agent chromogène est un colorant de type triphénylméthane, de type fluorane, de type azaphtalide et/ou de type fluorène, de manière préférée de type fluorane.

11. Matériau d'enregistrement thermosensible selon au moins l'une quelconque des revendications 8 à 10, dans lequel est présent, outre le révélateur chromogène non phénolique, au moins un révélateur chromogène de la formule générale
Ar¹SO₂NH-C₆H₄SO₂-C₆H₄-NH-CO-NH-Ar²,
dans lequel Ar¹ et Ar² sont un radical phényle non substitué ou substitué.

12. Matériau d'enregistrement thermosensible selon la revendication 11, **caractérisé en ce que** Ar¹ est un radical phényle, et/ou que Ar² est un radical phényle.

13. Matériau d'enregistrement thermosensible selon la revendication 11 ou 12, **caractérisé en ce que** Ar¹ est substitué par au moins un radical alkyle C₁-C₅, un radical alcényle, un radical alcynyle, un radical benzyle, un radical formyle, un radical CN, un radical halogène, un radical NO₂₋, un radical RO-, un radical R-CO-, un radical RO₂C-, un radical R-OCO-, un radical R-SO₂-O, un radical R-O-SO₂, un radical R-SO₂-NH, un radical R-NH-SO₂₋, un radical R-NH-CO ou un radical R-CO-NH, dans lequel R est un radical alkyle en C₁-C₅, un radical alcényle, un radical alcynyle, un radical phényle, un radical tolyle ou un radical benzyle.

14. Procédé de fabrication d'un matériau d'enregistrement thermosensible selon au moins l'une quelconque des revendications 8 à 13, dans lequel une suspension aqueuse contenant les matériaux de départ de la couche chromogène thermosensible est appliquée et séchée sur un substrat de support, dans lequel la suspension d'application aqueuse présente une teneur en matières sèches d'environ 20 à environ 75 % en poids, de manière préférée d'environ 30 à environ 50 % en poids, et est appliquée et séchée avec le procédé de revêtement en rideau à une vitesse de fonctionnement de la coucheuse d'au moins environ 400 m/min, de manière préférée d'au moins environ 1000 m/min, de manière très particulièrement préférée d'au moins environ 1500 m/min.

15. Matériau d'enregistrement thermosensible pouvant être obtenu selon le procédé selon la revendication 14.
